(19) Europäisches Patentamt
European Patent Office
Office européen des brevets

(11) EP 1 159 267 B1

(12) **EUROPEAN PATENT SPECIFICATION**

(45) Date of publication and mention of the grant of the patent:
**09.06.2004 Bulletin 2004/24**

(21) Application number: **00913549.2**

(22) Date of filing: **22.02.2000**

(51) Int Cl.[7]: **C07D 209/08**, C07D 209/12, A61K 31/404, A61P 5/32

(86) International application number:
**PCT/US2000/004343**

(87) International publication number:
**WO 2000/051982 (08.09.2000 Gazette 2000/36)**

(54) **N-SUBSTITUTED INDOLINES AS ESTROGENIC AGENTS**

N-SUBSTITUIERTE INDOLINE ALS ÖSTROGENE MITTEL

INDOLINES N-SUBSTITUEES COMME AGENTS OESTROGENES

(84) Designated Contracting States:
**AT BE CH CY DE DK ES FI FR GB GR IE IT LI LU MC NL PT SE**
Designated Extension States:
**AL LT LV RO SI**

(30) Priority: **04.03.1999 US 262569**

(43) Date of publication of application:
**05.12.2001 Bulletin 2001/49**

(73) Proprietor: **Wyeth**
**Madison, New Jersey 07940-0874 (US)**

(72) Inventor: **ULLRICH, John, William**
**Schwenksville, PA 19473 (US)**

(74) Representative: **Wileman, David Francis, Dr.**
**c/o Wyeth Laboratories**
**Huntercombe Lane South**
**Taplow Maidenhead Berkshire SL6 OPH (GB)**

(56) References cited:
**EP-A- 0 802 183**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 159 267 B1

## Description

The present invention relates to new N-substituted indoline compounds which are useful as estrogenic agents, as well as pharmaceutical compositions, methods of treatment utilizing these compounds and processes for preparing them.

## Background of the Invention

Estrogen replacement therapy has been well established as the treatment of choice in women for the prevention of osteoporosis. [C. Christiansen, R. Lindsay, *Estrogen, Bone Loss and Preservation,* **Osteoporosis International, 1,** 15-21 (1990)] The problem with unopposed estrogen therapy is that proliferative effects on the uterus may occur and be associated with endometriosis and/or endometrial cancer. Although less clear, unopposed estrogen replacement therapy has been implicated in increasing the incidence of breast tumor formation. Non-steroidal antiestrogen drugs such as Tamoxifen have been used in the treatment of breast cancer. Tamoxifen has been shown to exert an estrogen-like effect on bone in humans while acting as an antagonist in uterine tissue. However, demonstration of partial agonistic effects in the uterus is of some concern. A recent antiestrogen drug, Raloxifene, Lilly's benzothiophene, is a non-steroidal antiestrogen which appears to be more tissue selective. While possessing the ability to spare bone, it has been demonstrated to stimulate uterine growth in animal models to a lesser degree than Tamoxifen. A review on the tissue selective action of estrogen receptor modulators has recently appeared. [T.A. Grese and J.A. Dodge In "Ann. Rep. in Med. Chem." J.A. Bristol, Ed. Academic Press, New York, 1996, p.181]

The use of indoles as estrogen antagonists has been reported by Von Angerer, Chemical Abstracts, Vol. 99, No. 7 (1983), Abstract No. 53886u. Also, see, J. Med. Chem. 1990, 33, 2635-2640; J. Med. Chem. 1987, 30, 131-136. Also see Ger. Offen., DE 3821148 A1 891228 and WO 96/03375.

EP-A-0 802 183 describes 2-phenyl-1-[4-(2-aminoethoxy)-benzyl]-indole compounds, which are useful as selective estrogen agonists.

The compounds described in the present invention possess moderate binding to the estrogen receptor (ER) and have potential use in treating osteoporosis, prostatic hypertrophy, breast cancer and endometrial cancer.

## Description of the Invention

N-substituted indolines of this invention are tissue-selective estrogen agonists/antagonists useful for the treatment of diseases associated with estrogen deficiency. They include compounds of the formula:

(I)

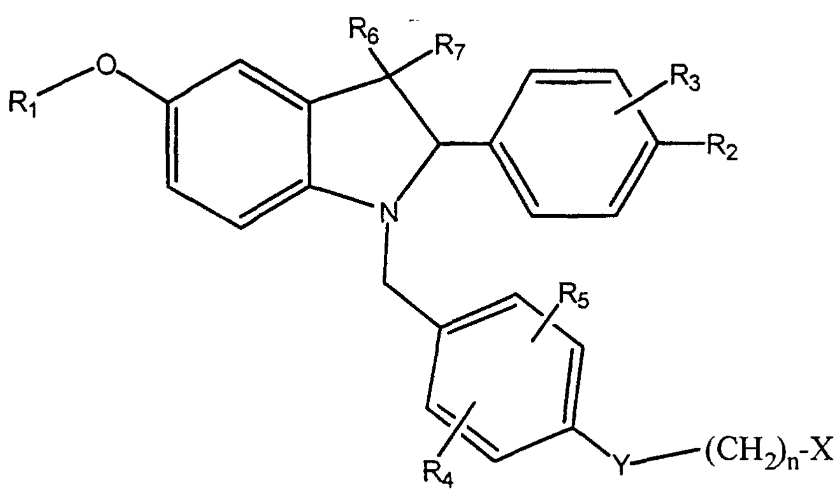

(I)

wherein:

$R_1$ is H or benzyl;
$R_2$ is H, -OH, or -O-benzyl;
$R_3$, $R_4$, and $R_5$ are independently selected from H, halogen, cyano, $C_1$-$C_6$ alkyl (straight chain or branched), trifluoromethyl, -OH or the $C_1$-$C_{12}$ esters (straight chain or branched) or $C_1$-$C_{12}$ alkyl ethers (straight chain or branched or cyclic) thereof, or $C_1$-$C_6$ halogenated ethers, preferably $C_1$-$C_3$ halogenated ethers, including trifluoromethyl ether and trichloromethyl ether;
$R_6$ is H or $C_1$-$C_6$ alkyl;
$R_7$ is $C_1$-$C_6$ alkyl;
n is 2 to 3;
Y is O or S; and
X is

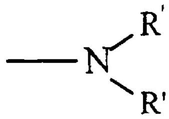

wherein R' is selected from $C_1$-$C_6$ lower alkyl the same or different, or X is cyclic moiety selected from:

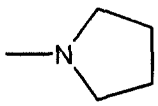

, 

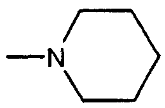

, 

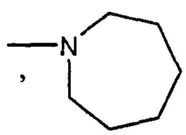

and

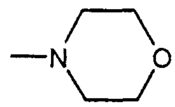

;

or a pharmaceutically acceptable salt thereof.

Examples of $C_1$-$C_{12}$ esters of the OH group are $C_2$-$C_{12}$ alkyl ester such as -OC(=O)$C_1$-$C_6$ alkyl esters.
Examples of alkyl groups are methyl, ethyl, n-propyl, isopropyl and n-butyl.
Examples of $R_6$ and $R_7$ are methyl.

Among the more preferred compounds of this invention are those in which Y is O. Preferably n is 2. Preferably $R_3$, $R_4$, and $R_5$ are independently selected from H, OH or halogen. It will be understood that the number of carbons in the alkyl groups of moieties $R_6$ and $R_7$ may be selected independently of each other.

The invention includes acceptable salt forms formed from the addition reaction with either inorganic or organic acids. Inorganic acids such as hydrochloric acid, hydrobromic acid, hydroiodic acid, sulfuric acid, phosphoric acid, nitric acid as well as organic acids such as acetic acid, propionic acid, citric acid, maleic acid, malic acid, tartaric acid, phthalic acid, succinic acid, methanesulfonic acid, toluenesulfonic acid, napthalenesulfonic acid, camphorsulfonic acid, benzenesulfonic acid are useful. It is known that compounds possessing a basic nitrogen can be complexed with many different acids (both protic and not protic) and usually it is preferred to administer a compound of this invention in the form of an acid addition salt. Additionally, this invention includes quaternary ammonium salts of the compounds herein, which can be prepared by reacting the nucleophilic amines of the side chain with a suitably reactive alkylating agent such as an alkyl halide or benzyl halide.

The compounds of this invention possess at least one one asymmetric centre and accordingly the compounds may exist and be isolated in a number of optically active stereoisomeric forms. This invention encompasses the compounds of formula I in any optically active form or mixtures thereof eg, racemates. Standard separation techniques may be used to isolate particular enantiomeric or diastereomeric forms. For example a racemic mixture may be converted to a mixture of optically active diastereoisomers by reaction with a single enantiomer of a 'resolving agent' (for example by diastereomeric salt formation or formation of a covalent bond). The resulting mixture of optically active diastereoisomers may be separated by standard techniques (e.g crystallisation or chromatography) and individual optically active diastereoisomers then treated to remove the 'resolving agent' thereby releasing the single enantiomer of the compound of the invention. Chiral chromatography (using a chiral support, eluent or ion pairing agent) may also be used to separate enantiomeric mixtures directly.

The compounds of the invention are partial estrogen agonists and display high affinity for the estrogen receptor. Unlike many estrogens, however, many of these compounds do not cause increases in uterine wet weight. These compounds are antiestrogenic in the uterus and can completely antagonize the trophic effects of estrogen agonists in uterine tissue. Due to the tissue selective nature of these compounds, they are useful in treating or preventing in a mammal disease states or syndromes which are caused or associated with an estrogen deficiency or an excess of estrogen.

The present compounds have the ability to behave like estrogen agonists by lowering cholesterol and preventing bone loss. These compounds are useful for treating many maladies which result from estrogen excess or deficiency including osteoporosis, prostatic hypertrophy, male pattern baldness, ovarian cancer, infertility, breast cancer, endometrial cancer, cardiovascular disease, contraception, Alzheimer's disease, cognitive decline and other CNS disorders, as well as certain cancers, including melanoma, prostrate cancer, cancers of the colon, CNS cancers, among others. Additionally, these compounds can be used for hormone replacement therapy in post-menopausal women or in other estrogen deficiency states where estrogen supplementation would be beneficial.

The compounds of this invention may also be used in methods of treatment for bone loss, which may result from an imbalance in an individual's formation of new bone tissues and the resorption of older tissues, leading to a net loss of bone. Such bone depletion results in a range of individuals, particularly in post-menopausal women, women who have undergone hysterectomy, those receiving or who have received extended corticosteroid therapies, those experiencing gonadal dysgenesis, and those suffering from Cushing's syndrome. Special needs for bone replacement can also be addressed using these compounds in individuals with bone fractures, defective bone structures, and those receiving bone-related surgeries and/or the implantation of prosthesis. In addition to those problems described above, these compounds can be used in treatments for osteoarthritis, hypocalcemia, hypercalcemia, Paget's disease, osteomalacia, osteohalisteresis, multiple myeloma and other forms of cancer having deleterious effects on bone tissues. Methods of treating the maladies listed herein are understood to comprise administering to an individual in need of such treatment a pharmaceutically effective amount of one or more of the compounds of this invention or a pharmaceutically acceptable salt thereof. This invention also includes pharmaceutical compositions utilizing one or more of the present compounds, and/or the pharmaceutically acceptable salts thereof, along with one or more pharmaceutically acceptable carriers, excipients, etc.

It is understood that the dosage, regimen and mode of administration of these compounds will vary according to the malady and the individual being treated and will be subject to the judgement of the medical practitioner involved. It is preferred that the administration of one or more of the compounds herein begin at a low dose and be increased until the desired effects are achieved.

Effective administration of these compounds may be given at an effective dose of from about 0.1 mg/day to about 1,000 mg/day. Preferably, administration will be from about 10 mg/day to about 600 mg/day in a single dose or in two or more divided doses. Such doses may be administered in any manner useful in directing the active compounds herein to the recipient's bloodstream, including orally, parenterally (including intravenous, intraperitoneal and subcutaneous injections), and transdermally. For the purposes of this disclosure, transdermal administrations are understood to include all administrations across the surface of the body and the inner linings of bodily passages including epithelial and mucosal tissues. Such administrations may be carried out using the present compounds, or pharmaceutically acceptable salts thereof, in lotions, creams, foams, patches, suspensions, solutions, and suppositories (rectal and vaginal).

Oral formulations containing the active compounds of this invention may comprise any conventionally used oral forms, including tablets, capsules, buccal forms, troches, lozenges and oral liquids, suspensions or solutions. Capsules may contain mixtures of the active compound(s) with inert fillers and/or diluents such as the pharmaceutically acceptable starches (e.g. corn, potato or tapioca starch), sugars, artificial sweetening agents, powdered celluloses, such as crystalline and microcrystalline celluloses, flours, gelatins, gums, etc. Useful tablet formulations may be made by conventional compression, wet granulation or dry granulation methods and utilize pharmaceutically acceptable diluents, binding agents, lubricants, disintegrants, suspending or stabilizing agents, including, but not limited to, magnesium stearate, stearic acid, talc, sodium lauryl sulfate, microcrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Oral formulations herein may utilize standard delay or time release formulations to alter the absorption of the active compound(s). Suppository formulations may be made from traditional materials, including cocoa butter, with or without the addition of waxes to alter the suppository's melting point, and glycerin. Water soluble suppository bases, such as polyethylene glycols of various molecular weights, may also be used.

This invention also provides processes for preparing the compounds of formula (I) which processes comprise the following:

a) a) alkylating a compound of formula:

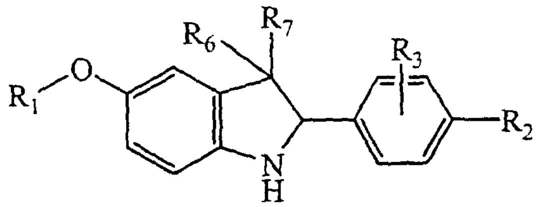

(II)

wherein $R_2$, $R_2$, $R_3$, $R_6$ and $R_7$ are as defined above, with a compound of formula:

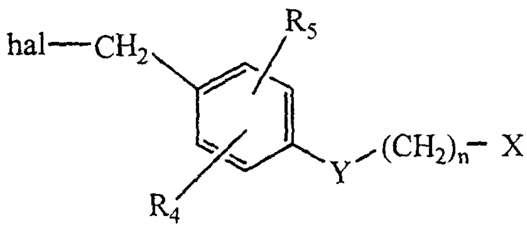

(III)

wherein n, $R_4$, $R_5$, Y and X are as defined above, to give a compound of formula I;
or
b) reacting a compound of formula

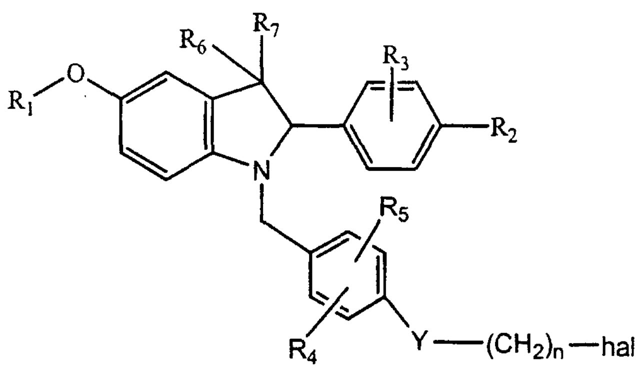

(IV)

wherein Y, $R_1$, $R_2$, $R_3$, $R_4$, $R_5$, $R_6$ and $R_7$ are as defined above and hal represents a halogen, e.g. chlorine or bromine, with an amine of formula:

H—X

(V)

wherein X is as defined above, to give a compound of formula (I),
or

c) debenzylating a compound of formula I wherein $R_1$ is optionally substituted benzyl and/or $R_2$ is optionally substituted benzyloxy, to give a compound of formula I wherein $R_1$ is hydrogen and/or $R_2$ is hydroxy,

or

e) esterifying a compound of formula I wherein at least one of $R_3$, $R_4$, or $R_5$ is hydroxy to an ester derivative thereof.

Methods for carrying out process a) → e) above are known in the art and/or are illustrated in the schemes below.

Compounds of this invention may be prepared by methods known in the art. For instance, the starting or core indoline can be prepared by the general methods of Schemes 1 and 2, below.

Scheme 1

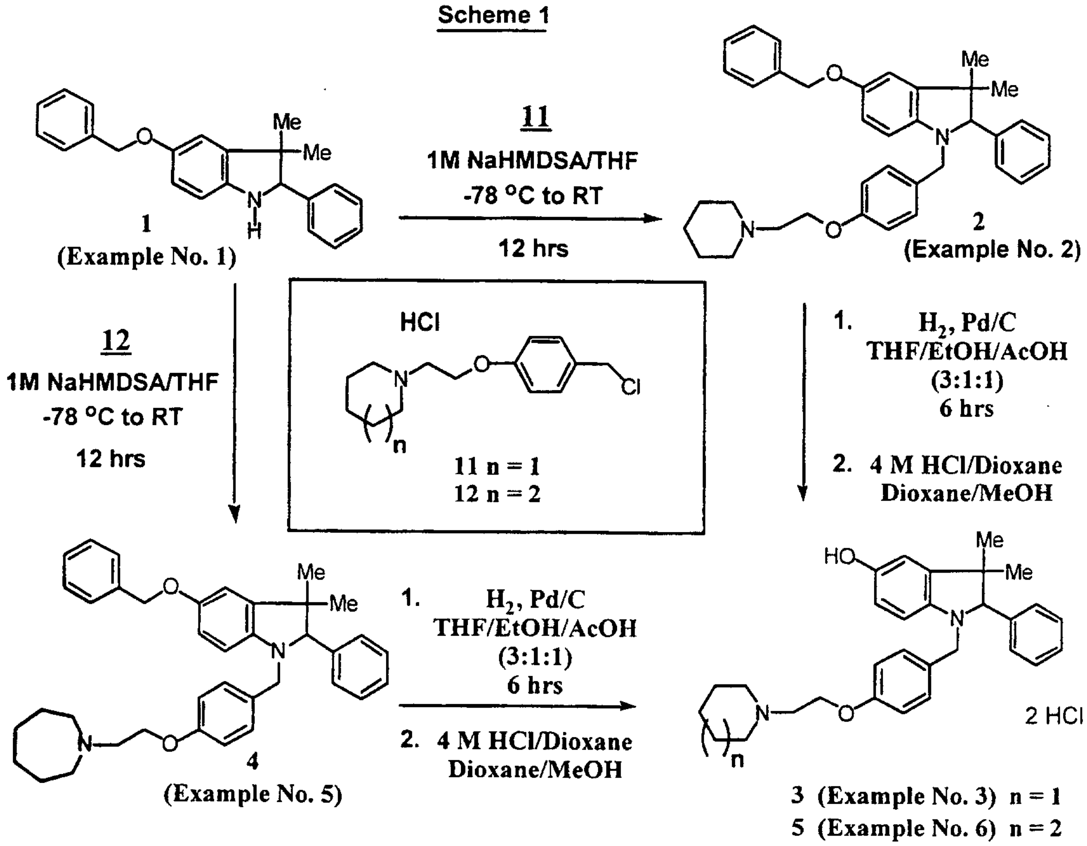


1 (Example No. 1)

2 (Example No. 2)

4 (Example No. 5)

3 (Example No. 3) n = 1

5 (Example No. 6) n = 2

Scheme 2

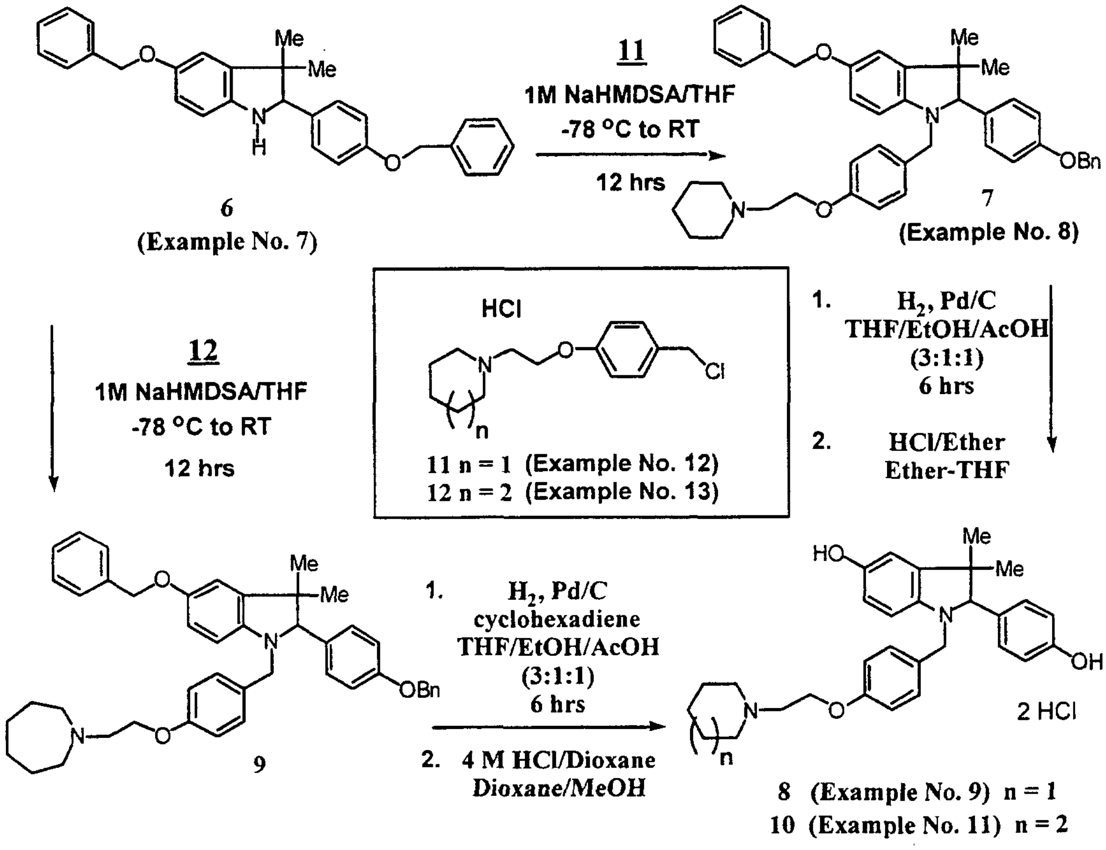


The synthesis of the compounds in this invention may be accomplished by deprotonation then alkylation of the core template **1** or **6** using the sodium salt of hexamethyldisilyl amide and the desired side chain. (Schemes 1 and 2)

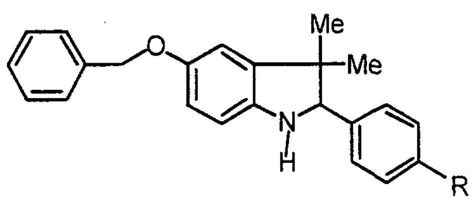

**1 R = H**
**6 R = OBn**

## Example No.1

## (Compound No. 1 in Scheme 1)

## 5-Benzyloxy-3,3-dimethyl-2-phenyl-2,3-dihydro-1H-indole

The protected dihydro-indoles **1** and **6**, shown above, were used as the core template to synthesize compounds **3** (Example No. 3), **5** (Example No. 6), **8** (Example No. 9) and 10 (Example No. 11). These material templates were prepared using the general method described by Letcher, R. M. et. al., J. Chem. Soc. Perkin Trans., 1993, Vol. 1, pp. 939 - 944. For example, Template **1** was prepared from 4-benzyloxyphenyl hydrazine and isobutyrophenone.

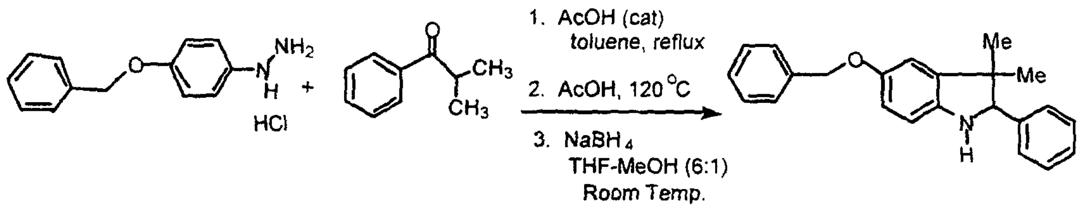


To a solution of 4-benzyloxy-phenyl hydrazine HCl (2.5 g, 11.7 mmol) and isobutyrophenone (2.1 g, 14.0 mmol) in 20 ml toluene was added acetic acid (cat) and the resulting solution was refluxed with azeotropic removal of $H_2O$ for 14 hours. The reaction mixture was cooled to room temperature and concentrated. The resulting semi-solid was taken up in acetic acid and refluxed for 12 hours. The reaction mixture was cooled to room temperature and concentrated. The semi-solid residue was taken up in ether and neutralized with $K_2CO_3$. The ether layer was dried and concentrated. The resulting solid imine was dissolved in THF-MeOH (6:1), cooled to 0 C and $NaBH_4$ (0.53 g, 13.2 mmol) was added. The solution was allowed to warm to room temperature and stirred for 0.5 hours. The reaction mixture was poured into 15% HCl and made basic with $K_2CO_3$. The organic layer was separated, washed with brine, dried over $NaSO_4$, and concentrated. The crude product was purified by column chromatography ($SiO_2$, hexane-ethyl acetate (9:1). The desired template, **1**, was isolated as an orange semi-solid (2.9 g, 75%): $^1H$ NMR ($CDCl_3$) δ d 0.7 (s, 3H), 1.4 (s, 3H), 3.85 (br.s. 1H), 4.8 (s. 1H), 5.0 (s, 2H), 6.62 (d, 1H), 6.67 (s, 1H), 6.8 (dd, 1H), 7.3-7.5 (m, 10H); $^{13}C$-NMR ($CDCl_3$) δ 24.3 (q), 26.3 (q), 45.5 (s), 71.0 (t), 74.9 (d), 110.4 (d), 110.9 (d), 113.0 (d), 127.4, 127.5 127.6, 127.8, 128, 128.5 (d), 137.6 (s), 139.7 (s), 139.9 (s), 143.4 (s), 152.9 (s); IR (film) 3390, 3040, 2980, 1490, 1050 $cm^{-1}$; mass spectrum m/e 330 (M+1);
CHN calc. for $C_{23}H_{23}NO$

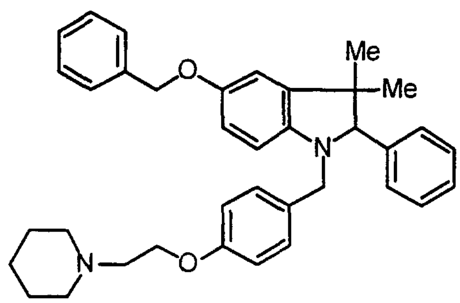

**2**

**Example No. 2**

**(Compound No. 2 in Scheme 1)**

**5-Benzyloxy-3,3-dimethyl-2-phenyl-1-[4-(2-piperidin-1-yl-ethoxy)-benzyl]-2,3-dihydro-1H-indole**

To a solution of 5-benzyloxy-indoline **1** (0.2 g, 0.61 mmol) and piperidine ethoxy benzylbromide **11** (0.18 g, 0.61 mmol) in 3 mL THF at -78 °C was added 1.53 mL, (1.53 mmol, 1M, NaHMDSA-THF, 2.5 eq.). The reaction mixture was allowed to warm to room temperature and stirred for 12 hrs. The reaction was then poured into water, extracted with EtOAc. The organic layer was dried with $MgSO_4$ and concentrated. The product was purified by flash chromatography (15% EtOAc-Hexane) to give the desired N-alkylated indoline **2** as a yellow foam.
$^1H$-NMR ($CDCl_3$) δ 0.79 (s, 3H), 1.34 (s, 3H), 1.4 (m, 2H), 1.6 (m, 4H), 2.5 (m, 4H), 2.8 (t, 2H), 3.82 (d, AB q, 1H), 4.1 (t, 2H), 4.2 (s, 1H), 4.25 (d, AB q, 1H), 5.0 (s, 1H), 6.33 (d, 1H), 6.6 (dd, 1H), 6.7 (dd, 1H), 6.8 (d, 2H), 7.2 (d, 2H), 7.3-7.45 (m, 10H); $^{13}C$-NMR ($CDCl_3$) δ 24.18(t), 25.05 (q), 25.9 (t), 26.0 (q), 44.5 (s), 51.5, 55.0, 57.9, 65.9, 70.9 (t), 80.5 (d), 109.0, 110.7, 112.5, 114.4, 127.53, 127.55, 127.7, 128.1, 128.5, 128.6 (d), 130.6, 137.6, 137.7, 140.4, 145.3, 152.6, 157.72 (s); mass spectrum m/e 547 (M+1).

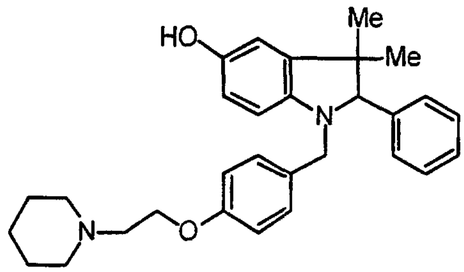

**3**

**Example No. 3**

**(Compound No. 3 in Scheme 1)**

**3,3-dimethyl-2-phenyl-1-[4-(2-piperidin-1-yl-ethoxy)-benzyl] -2,3-dihydro-1H-indol-5-ol**

To a solution of the N-alkylated indoline **2** (0.14 g, 0.26 mmol) in 8 mL (THF-EtOH-AcOH, 5:2:1) was added 0.14 g (10% Pd/C) followed by 0.25 mL cyclohexadiene (10 eq.) The reaction mixture was stirred at RT. for 7 hrs. The reaction mixture was filtered through celite, concentrated. The residue was taken up in EtOAc, washed with $NaHCO_3$. The organic layer was dried with $MgSO_4$ and concentrated. The product was purified by flash chromatography (8% MeOH-Methylene chloride) to give the desired phenol-indoline **3**.
$^1$H-NMR ($CDCl_3$) $\delta$ 0.65 (s, 3H), 1.25 (s, 3H), 1.3-1.5 (m, 6H), 2.4 (m, 6H), 2.65 (m, 2H), 3.7 (d, AB q, 1H), 4.1 (m, 1H), 4.25 (d, AB q, 1H), 6.28 (d, 1H), 6.4 (dd, 1H), 6.5(S,1H), 6.8(d, 2H), 7.1(d, 2H), 7.3-7.45 (m, 5H), 8.6 (S,1H); mass spectrum m/e 457 (M+1).

**Example No. 4**

**(Compound No. 3 (HCl Salt) in Scheme 1)**

**3,3-dimethyl-2-phenyl-1-[4-(2-piperidin-1-yl-ethoxy)-benzyl]-2,3-dihydro-1H-indol-5-ol (HCl)**

A solution of the phenol indoline **3** (0.043 g, 0.11 mmol) in 2 mL of $Et_2O$-THF (4-1) is treated with 1.2 eq (1M HCl/ $Et_2O$). The reaction mixture is then stirred for 2 hours, concentrated giving the desired HCl salt.

**Example No. 5**

**(Compound No. 4 in Scheme 1)**

**1-[4-(2-Azepan-1-yl-ethoxy)-benzyl]-5-benzyloxy-3,3-dimethyl-2-phenyl-2,3-dihydro-1H-indole**

Prepared as described above for compound **2.**
$^1$H-NMR ($CDCl_3$) $\delta$ 0.8 (s.3H), 1.35 (s. 3H), 1.65 (m, 8H), 2.8 (m, 9H), 3.0 (t. 2H), 3.8 (A-B q, 1H), 4.1 (t. 2H), 4.2 (s, 1H), 4.3 (AB q, 1H), 4.95 (s, 2H), 6.35 (d, 1H), 6.65 (dd, 1H), 7.5 (s, 1H), 6.85 (d. 2H), 7.2 (d, 2H), 7.25-7.5 (m, 10H); mass spectrum m/e 561 (M+1).

**Example No. 6**

**(Compound No. 5 in Scheme 1)**

**1-[4-(2-Azepan-1-yl-ethoxy)-benzyl]-3,3-dimethyl-2-phenyl-2,3-dihydro-1H-indol-5-ol**

Prepared as described above for compound **3.**
$^1$H-NMR (DMSO-$d_6$) $\delta$ 0.6 (5, 3H), 1.15 (S, 3H), 1.4 (m, 8H), 2.6 (m, 4H), 2.7 (m, 2H), 3.55 (ABq, 1H), 3.8 (7, 2H), 3.9 (S, 1H), 4.05 (ABq, 1H), 6.15 (d, 1H), 6.25 (d, 1H), 6.35 (S, 1H), 6.7 (D, 2H), 7.0 (d, 2H), 7.2-7.4 (m, 5H), 8.5 (S, 1H);

mass spectrum m/e 471 (M+1).

**Example No. 7**

**(Compound No. 6 in Scheme 2)**

**5-Benzyloxy-2-(4-benzyloxy-phenyl)-3,3-dimethyl-2,3-dihydro-1H-indole**

The title compound was prepared using the general method described by Letcher, R. M. et. al., J. Chem. Soc. Perkin Trans., 1993, Vol. 1, pp. 939 - 944. (See Example No. 1, above) from 4-benzyloxyphenyl hydrazine and (4-benzyloxy)-phenyl isopropyl ketone.
$^1$H-NMR ($CDCl_3$) δ 0.7 (s, 3H), 1.35 (s, 3H), 3.85 (br. s, 1H), 4.5 (S, 1H), 5.0 (s, 2H, 5.1 (s, 2H), 6.6 (d, 1H), 6.7 (s, 1H), 6.75 (s, 1H), 6.95 (d, 2H), 7.25-7.5 (m, 12H).
$^{13}$C-NMR ($CDCl_3$) d 24.2, 26.2 (q), 45.5 (s), 70.0, 71.0 (t), 74.5 (d), 109.4, 110.9, 112.9, 114.3, 127.5, 127.6, 127.8, 127.9, 128.5, 128.6, (d), 132.2, 137.04, 137.6, 139.8, 143.4, 152.9, 158.3 (s); mass spectrum m/e 436 (M+1).

**Example No. 8**

**(Compound No. 7 in Scheme 2)**

**5-Benzyloxy-2-(4-benzyloxy-phenyl)-3,3-dimethyl-1-[4-(2-piperidin-1-yl-ethoxy)-benzyl]2,3-dihydro-1H-indole**

Prepared as described above for compound **2.**
$^1$H-NMR ($CDCl_3$) δ 0.75 (s, 3H), 1.2 (s, 3H), 1.6-1.8 (m, 6H), 2.75 (t.2H), 3.2-3.85 (m, 4H), 4.05 (t.2H), 4.7 (br.s, 1H), 4.35 (br. s 2H), 5.0 (s, 2H), 5.1 (s, 2H), 6.3 (d. 2H), 6.5 (m, 2H), 6.6 (d, 2H), 6.85 (d. 2H), 6.95 (d, 2H), 7.05 (d, 2H), 7.2-7.5 (m, 8H), 7.5 (br. s. 1H).

**Example No. 9**

**(Compound No. 8 in Scheme 2)**

**2-(4-Hydroxy-phenyl)-3,3-dimethyl-1-[4-(2-piperidin-1-yl-ethoxy)-benzyl]-2,3-dihydro-1H-indol-5-ol**

Prepared as described above for compound **3.**
$^1$H-NMR (DMSO) δ 0.75 (s, 3H), 1.2 (s, 3H), 1.6 - 1.8 (m, 6H), 2.75 (t.2H), 3.2-3.85 (m, 4H), 4.05 (t.2H), 4.7 (br.s, 1H), 4.35 (br. s 2H), 6.3 (d. 2H), 6.5 (m, 2H), 6.6 (d, 2H), 6.85 (d. 2H), 7.05 (d, 2H), 7.5 (br. s. 1H); mass spectrum m/e 473 (M+1).

**Example No. 10**

**(Compound No. 9 in Scheme 2)**

**5-Benzyloxy-2-(4-benzyloxy-phenyl)-3,3-dimethyl-1-[4-(2-Azepan-1-yl-ethoxy)-benzyl]-2,3-dihydro-1H-indole**

Prepared as described above for compound **2.**
$^1$H-NMR ($CDCl_3$) δ 0.8 (s, 3H), 1.35 (s, 3H), 1.6-1.8 (m, 6H), 2.8 (br.s, 4H) 3.1 (t, 2H), 3.8 (AB q, 1H), 4.1 (t. 3H), 4.25 (AB q. 1H), 4.95 (s, 2H), 5.1 (s, 2H), 6.3 (d, 1H), 6.6 (d, 1M), 6.75 (s, 1H), 6.8 (d, 2H), 6.95 (m, 3H), 7.1 (d, 1H), 7.2 (d, 2H), 7.3 (m, 8H), 7.55 (d, 1H), 8.1 (d, 1H).

**Example No. 11**

**(Compound No. 10 in Scheme 2):**

**1-[4-(2-Azepan-1-yl-ethoxy)-benzyl]-2-(4-hydroxy-phenyl)-3,3-dimethyl-2-Phenyl-2,3-dihydro-1H-indol-5-ol**

Prepared as described above for compound **3**.
$^1$H-NMR ($CDCl_3$) δ 0.8 (s, 3H), 1.15 (s, 3H), 1.6 (t, 2H), 1.7 (m, 4H), 3.1 (m, 2H), 3.3-3.5 (m, 4H), 4.2 (t, 2H), 4.3-4.5 (m 2H), 6.7 (d, 4H), 6.8 (d, 4H) 6.9 (d, 2H), 2.1 (d, 2H), 7.3 (br. S, 1H).

**Example No. 12**

**(Compound No. 11 in Scheme 2)**

**1-[2-(4-Chloromethyl-phenoxy)-ethyl]-piperidine**

This material is prepared from the amino alcohol 1-[2-(4-Hydroxymethyl-phenoxy)-ethyl]piperidine by treatment with thionyl chloride in THF at 0°C. Resulting solid is used without further purification.
$^1$H-NMR ($CDCl_3$) δ 1.7-1.9 (m, 6H), 2.5 (br. s, 4H), 2.7 (t, 2H), 4.1 (t, 2H), 4.6 (s, 2H), 6.9 (d, 2H), 7.3 (d, 2H). 12.1 (br s, 1H).

**Example No. 13**

**(Compound No. 12 in Scheme 2)**

**1-[2-(4-Chloromethyl-phenoxy)-ethyl]-azepane**

This material is prepared from the amino alcohol 1-[2-(4-Hydroxymethyl-phenoxy)-ethyl]azapine by treatment with thionyl chloride in THF at 0°C. Resulting solid is used without further purification.
$^1$H-NMR ($CDCl_3$) δ 1.7 (m, 2H), 1.9 (m, 4H), 2.2 (m, 2H), 3.1 (m, 2H), 3.4 (7, 2H), 3.6 (t.2H), 6.9 (d, 2H), 7.3 (d, 2H), 12.5 (1H).

**Receptor Binding Assay**

**Objective:** To identify compounds that compete with 17β-estradiol for estrogen receptor (ER) binding. The widely accepted mode for estrogenic action is via its high affinity receptor protein. Compounds which demonstrate an ability to bind to the ER may then regulate physiological processes associated with estrogen action.

**Procedure:** Receptor Preparation: CHO cells overexpressing the estrogen receptor are grown in 150 $mm^2$ dishes in DMEM+ 10% dextran coated charcoal, stripped fetal bovine serum. The plates are washed twice with PBS and once with 10 mM Tris-HCl, pH 7.4, 1 mM EDTA. Cells are harvested by scraping the surface and then the cell suspension is placed on ice. Cells are disrupted with a hand-held motorized tissue grinder using two, 10-second bursts. The crude preparation is centrifuged at 12,000 x g for 20 min. followed by a 60 min spin at 100,000 x g to produce a ribosome-free cytosol. The cytosol is frozen and stored at -80°C. Protein concentration of the cytosol is estimated using the BCA assay with BSA as the reference standard protein.

**Binding Assay conditions:**

The competition assay is performed in a 96-well plate (polystyrene*) which binds <2.0% of the total input [3H]-17β-estradiol. Each data point is gathered in triplicate. 100 ug/100 ul of the receptor preparation is aliquoted per well. A saturating dose of 2.5 nM [3H]17 β-estradiol + competitor (or buffer) in a 50 μl volume is added in the preliminary competition when 100x and 500x competitor concentrations are evaluated. For an $IC_{50}$ determination, where 12 concentrations of competitor are evaluated, only 0.8 nM [3H]17β-estradiol is used. The plate is incubated at room temperature for 2.5 h. At the end of this incubation period 150 μl of ice-cold dextran coated charcoal (5% activated charcoal coated with 0.05% 69K dextran) is added/well and the plate is immediately centrifuged at 900 x g for 5 minutes at 4°C. 200μl of the supernatant solution is removed for scintillation counting. Samples are counted to 2% or 10 min, whichever occurs first.

*Because polystyrene absorbs a small amount of [3H]17β-estradiol, wells containing radioactivity and cytosol, but not processed with charcoal, are included to quantitate amount of available isotope. Also, wells containing radioactivity but no cytosol are processed with charcoal to estimate unremovable DPM of [3H]17β-estradiol. Corning #25880-96 96-well plates were used because, among those tested, they have demonstrated to bind the least amount of estradiol.

**Analysis of Results:**

Counts per minute (CPM) of radioactivity are automatically converted to disintegrations per minute (DPM) by the Beckman LS7500 Scintillation Counter using a set of quenched standards to generate a H# for each sample. To calculate the % of estradiol binding in the presence of 100 or 500 fold competitor the following formula is applied:

((DPM sample-DPM not removed by charcoal/(DPM estradiol-DPM not

removed by charcoal)) x 100% = % of estradiol binding

For the generation of $IC_{50}$ curves, % binding is plotted vs [compound]. $IC_{50}$'s are generated for compounds that show >10% competition at up to a 500x competitor concentration.

**Reference Compounds:**

Reference compounds and those of Example Nos. 4, 6, 9 and 11 have been evaluated and their $IC_{50}$ concentration determined. The concentration of these compounds required to displace 50% of [3H]17β-estradiol is:

| | |
|---|---|
| estradiol: 0.08 μM | Example No. 4: 0.8 μM |
| tamoxifen: 4.50 μM | Example No. 6: 1.0 μM |
| raloxifene 0.04 μM | Example No. 9: 0.31 μM |
| 17α-dihydroequilin 0.15 μM | Example No. 11: 0.35 μM |

## Claims

**1.** A compound of the formula:

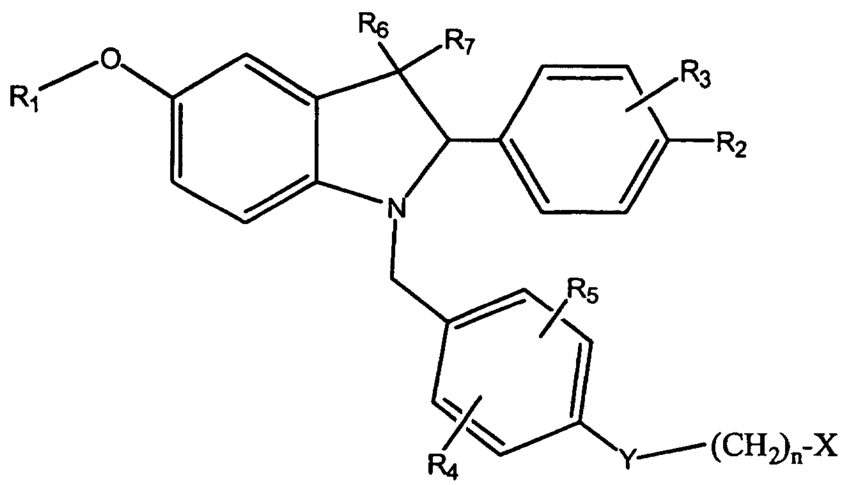

(I)

wherein:

$R_1$ is H or benzyl;
$R_2$ is H, -OH, or -O-benzyl;
$R_3$, $R_4$, and $R_5$ are independently selected from H, halogen, cyano, $C_1$-$C_6$ alkyl (straight chain or branched), trifluoromethyl, -OH or the $C_1$-$C_{12}$ esters (straight chain or branched) or $C_1$-$C_{12}$ alkyl ethers (straight chain or branched or cyclic) thereof, or $C_1$-$C_6$ halogenated ethers;
$R_6$ is H or $C_1$-$C_6$ alkyl;
$R_7$ is $C_1$-$C_6$ alkyl;
n is 2 to 3;

Y is O or S; and
X is

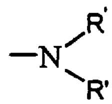

wherein R' is selected from $C_1$-$C_6$ lower alkyl the same or different or X is a moiety selected from:

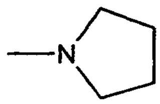 , 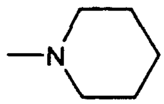 , 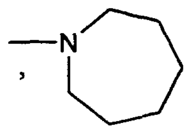 and 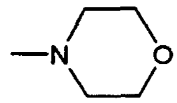 ;

or a pharmaceutically acceptable salt thereof.

**2.** A compound as claimed in Claim 1 wherein Y is O or a pharmaceutically acceptable salt thereof.

**3.** A compound as claimed in Claim 1 or Claim 2 wherein n is 2 and $R_3$, $R_4$, and $R_5$ are independently selected from H, OH or halogen; or a pharmaceutically acceptable salt thereof.

**4.** A compound of Claim I which is one of the following:

5-benzyloxy-3,3-dimethyl-2-phenyl-1-[4-(2-piperidin-1-yl-ethoxy)-benzyl]-2,3-dihydro-1H-indole;
3,3-dimethyl-2-phenyl-1-[4-(2-piperidin-1-yl-ethoxy)-benzyl]-2,3-dihydro-1H-indol-5-ol;
1-[4-(2-azepan-1-yl-ethoxy)-benzyl]-5-benzyloxy-3,3-dimethyl-2-phenyl-2,3-dihydro-1H-indole;
1-[4-(2-azepan-1-yl-ethoxy)-benzyl]-3,3-dimethyl-2-phenyl-2,3-dihydro-1H-indol-5-ol;
5-benzyloxy-2-(4-benayloxy-phenyl)-3,3-dimethyl-1-[4-(2-piperidin-1-yl-ethoxy)-benzyl]2,3-dihydro-1H-indole;
2-(4-hydroxy-phenyl)-3,3-dimethyl-1-[4-(2-piperidin-1-yl-ethoxy)-benzyl]-2,3-dihydro-1H-indol-5-ol;
5-benzyloxy-2-(4-benzyloxy-phenyl)-3,3-dimethyl-1-[4-(2-Azepan-1-yl-ethoxy)-benzyl]-2,3 -dihydro-1H-indole;

or

1-[4-(2-azepan-1-yl-ethoxy)-benzyl]-2-(4-hydroxy-phenyl)-3,3-dimethyl-2-phenyl-2,3-dihydro-1H-indol-5-ol;

or a pharmaceutically acceptable salt thereof.

**5.** A pharmaceutical composition comprising a compound of any one of Claims 1 to 4 or a pharmaceutically acceptable salt thereof, and a pharmaceutically acceptable carrier or excipient.

**6.** Use of a compound as claimed in any one of Claims 1 to 4 in the preparation of a medicament for treating or preventing bone loss in a mammal, the method comprising administering to a mammal.

**7.** Use of a compound as claimed in any one of Claims 1 to 4 in the preparation of a medicament for treating or preventing disease states or syndromes which are caused or associated with an estrogen deficiency in a mammal.

**8.** Use of a compound as claimed in any one of Claims 1 to 4 in the preparation of a medicament for treating or preventing cardiovascular disease in a mammal, the method comprising administering to a mammal.

**9.** A process for preparing a compound of formula I as claimed in Claim 1 or a pharmaceutically acceptable salt thereof which comprises one of the following:

a) alkylating a compound of formula:

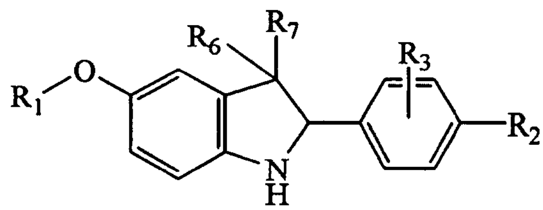

(II)

wherein $R_2$, $R_2$, $R_3$, $R_6$ and $R_7$ are as defined in claim 1, with a compound of formula:

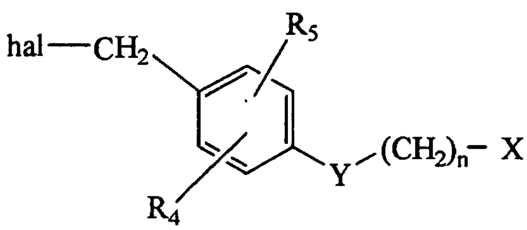

(III)

wherein n, $R_4$, $R_5$, Y and X are as defined in claim 1, to give a compound of formula I; or
b) reacting a compound of formula

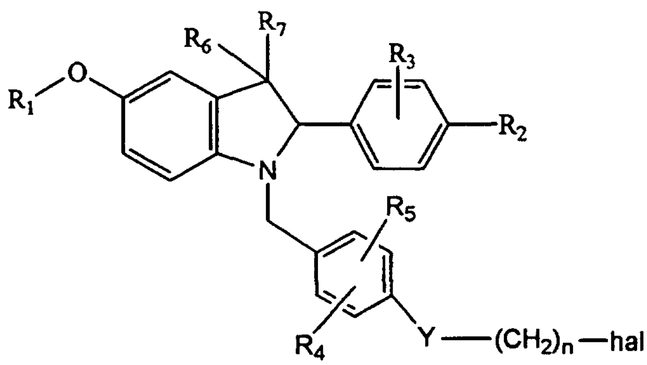

(IV)

wherein Y, $R_1$, $R_2$, $R_3$, $R_4$, $R_5$, $R_6$ and $R_7$ are as defined in claim 1 and hal represents a halogen, e.g. chlorine or bromine, with an amine of formula:

H-X

(V)

wherein X is as defined above, to give a compound of formula I,

or

c) debenzylating a compound of formula I wherein $R_1$ is optionally substituted benzyl and/or $R_2$ is optionally substituted benzyloxy, to give a compound of formula I wherein $R_1$ is hydrogen and/or $R_2$ is hydroxy,

or

e) esterifying a compound of formula I wherein at least one of $R_3$, $R_4$, or $R_5$ is hydroxy to an ester derivative thereof.

**10.** A compound as claimed in any one of Claims 1 to 4 for use as a pharmaceutical.

**Patentansprüche**

**1.** Verbindung der Formel:

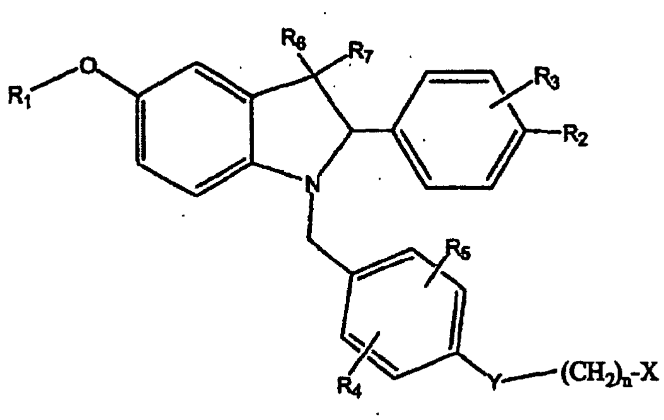

(I)

worin:

$R_1$ für H oder Benzyl steht;
$R_2$ für H, -OH oder -O-Benzyl steht;
$R_3$, $R_4$ und $R_5$ unabhängig ausgewählt werden aus H, Halogen, Cyano, $C_1$-$C_6$-Alkyl (geradkettig oder verzweigt), Trifluormethyl, -OH oder den $C_1$-$C_{12}$-Estern (geradkettig oder verzweigt) oder $C_1$-$C_{12}$-Alkylethern (geradkettig oder verzweigt oder cyclisch) davon, oder $C_1$-$C_6$-halogenierten Ethern;
$R_6$ für H oder $C_1$-$C_6$-Alkyl steht;
$R_7$ für $C_1$-$C_6$-Alkyl steht;
n 2 bis 3 ist;
Y für O oder S steht und
X für

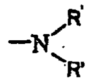

steht, worin R' ausgewählt wird aus $C_1$-$C_6$ nied.-Alkyl gleich oder unterschiedlich oder X für eine Komponente

steht, ausgewählt aus:

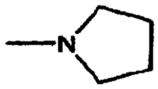 , 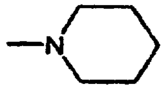 , 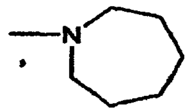 und 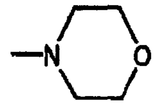 ;

oder ein pharmazeutisch annehmbares Salz davon.

**2.** Verbindung wie in Anspruch 1 beansprucht, worin Y für O steht, oder ein pharmazeutisch annehmbares Salz davon.

**3.** Verbindung wie in Anspruch 1 oder Anspruch 2 beansprucht, worin n 2 ist und $R_3$, $R_4$ und $R_5$ unabhängig ausgewählt werden aus H, OH oder Halogen; oder ein pharmazeutisch annehmbares Salz davon.

**4.** Verbindung nach Anspruch 1, welche eine der folgenden ist:

5-Benzyloxy-3,3-dimethyl-2-phenyl-1-[4-(2-piperidin-1-ylethoxy)-benzyl]-2,3-dihydro-1H-indol;
3,3-Dimethyl-2-phenyl-1-[4-(2-piperidin-1-yl-ethoxy)-benzyl]-2,3-dihydro-1H-indol-5-ol;
1-[4-(2-Azepan-1-yl-ethoxy)-benzyl]-5-benzyloxy-3,3-dimethyl-2-phenyl-2,3-dihydro-1H-indol;
1-[4-(2-Azepan-1-yl-ethoxy)-benzyl]-3,3-dimethyl-2-phenyl-2,3-dihydro-1H-indol-5-ol;
5-Benzyloxy-2-(4-benzyloxy-phenyl)-3,3-dimethyl-1-[4-(2-piperidin-1-yl-ethoxy)-benzyl]-2,3-dihydro-1H-indol;
2-(4-Hydroxy-phenyl)-3,3-dimethyl-1-[4-(2-piperidin-1-ylethoxy)-benzyl]-2,3-dihydro-1H-indol-5-ol;
5-Benzyloxy-2-(4-benzyloxy-phenyl)-3,3-dimethyl-1-[4-(2-azepan-1-yl-ethoxy)-benzyl]-2,3-dihydro-1H-indol;
oder
1-[4-(2-Azepan-1-yl-ethoxy)-benzyl]-2-(4-hydroxy-phenyl)-3,3-dimethyl-2-phenyl-2,3-dihydro-1H-indol-5-ol;

oder ein pharmazeutisch annehmbares Salz davon.

**5.** Pharmazeutische Zusammensetzung, welche eine Verbindung nach einem der Ansprüche 1 bis 4 oder ein pharmazeutisch annehmbares Salz davon und einen pharmazeutisch annehmbaren Träger oder Exzipienten umfasst.

**6.** Verwendung einer Verbindung wie in einem der Ansprüche 1 bis 4 beansprucht bei der Herstellung eines Medikaments zum Behandeln oder Verhindern von Knochenverlust in einem Säuger, wobei das Verfahren das Verabreichen an einen Säuger umfasst.

**7.** Verwendung einer Verbindung wie in einem der Ansprüche 1 bis 4 beansprucht bei der Herstellung eines Medikaments zum Behandeln oder Verhindern von Krankheitszuständen oder Syndromen, welche mit einem Estrogenmangel in einem Säuger in Verbindung gebracht oder davon verursacht werden.

**8.** Verwendung einer Verbindung wie in einem der Ansprüche 1 bis 4 beansprucht bei der Herstellung eines Medikaments zum Behandeln oder Verhindern von kardiovaskulärer Erkrankung in einem Säuger, wobei das Verfahren das Verabreichen an einen Säuger umfasst.

**9.** Verfahren zum Herstellen einer Verbindung der Formel I wie in Anspruch 1 beansprucht oder eines pharmazeutisch annehmbaren Salzes davon, welches eines der folgenden umfasst:

a) Alkylieren einer Verbindung der Formel:

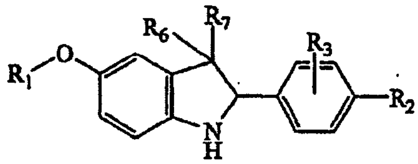

(II)

worin $R_1$, $R_2$, $R_3$, $R_6$ und $R_7$ wie in Anspruch 1 definiert sind, mit einer Verbindung der Formel:

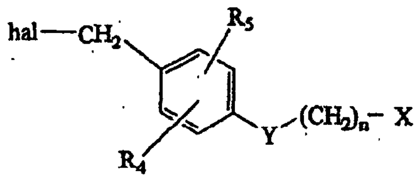

(III)

worin n, $R_4$, $R_5$, Y und X wie in Anspruch 1 definiert sind, um eine Verbindung der Formel I zu ergeben; oder
b) Umsetzen einer Verbindung der Formel

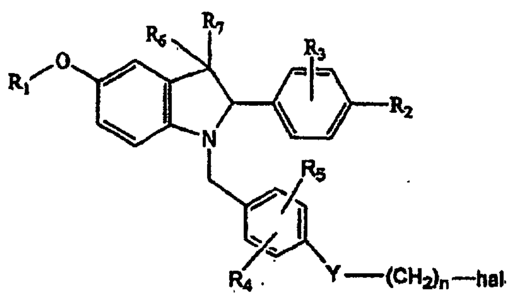

(IV)

worin Y, $R_1$, $R_2$, $R_3$, $R_4$, $R_5$, $R_6$ und $R_7$ wie in Anspruch 1 definiert sind und hal ein Halogen, z.B. Chlor oder Brom darstellt, mit einem Amin der Formel:

H-X,

(V),

worin X wie oben definiert ist, um eine Verbindung der Formel I zu ergeben,
oder
c) Debenzylieren einer Verbindung der Formel I, worin $R_1$ gegebenenfalls substituiertes Benzyl darstellt und/oder $R_2$ gegebenenfalls substituiertes Benzyloxy darstellt, um eine Verbindung der Formel I zu ergeben, worin

$R_1$ Wasserstoff darstellt und/oder $R_2$ Hydroxy darstellt, oder
e) Verestern einer Verbindung der Formel I, worin mindestens eines von $R_3$, $R_4$ oder $R_5$ für Hydroxy steht, in ein Esterderivat davon.

**10.** Verbindung wie in einem der Ansprüche 1 bis 4 beansprucht zur Verwendung als Pharmazeutikum.

### Revendications

**1.** Composé de formule : dans laquelle

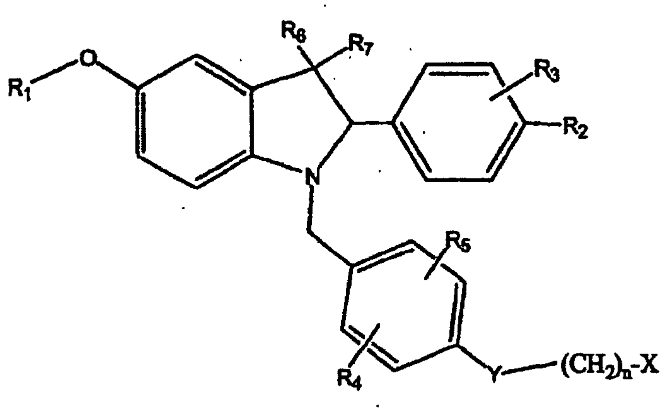

(I)

$R_1$ est H ou un benzyle;
$R_2$ est H, -OH ou -O-benzyle;
$R_3$, $R_4$ et $R_5$ sont indépendamment sélectionnés parmi H, un halogène, un cyano, un alkyle en $C_1$-$C_6$ (à chaîne linéaire ou ramifié), un trifluorométhyle, -OH ou des esters en $C_1$-$C_{12}$ (à chaîne linéaire ou ramifiés) ou des alkyléthers en $C_1$-$C_{12}$ (à chaîne linéaire ou ramifiés ou cycliques) de ceux-ci ou des éthers halogénés en $C_1$-$C_6$:
$R_6$ est H ou un alkyle en $C_1$-$C_6$;
$R_7$ est un alkyle en $C_1$-$C_6$;
n est 2 à 3;
Y est O ou S; et
X est

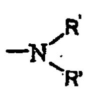

dans laquelle R' est sélectionné parmi un alkyle inférieur en $C_1$-$C_6$ identique ou différent ou X est une partie sélectionnée parmi :

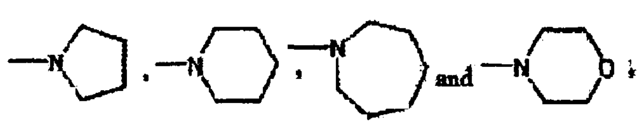

ou un sel pharmaceutiquement acceptable de celui-ci.

**2.** Composé suivant la revendication 1 dans lequel Y est O ou un sel pharmaceutiquement acceptable de celui-ci.

3. Composé suivant la revendication 1 ou la revendication 2 dans lequel n est 2 et $R_3$, $R_4$ et $R_5$ sont indépendamment sélectionnés parmi H, OH ou un halogène; ou un sel pharmaceutiquement acceptable de celui-ci.

4. Composé suivant la revendication 1 qui est un des suivants :

le 5-benzyloxy-3,3-diméthyl-2-phényl-1-[4-(2-pipéridin-1-yléthoxy)benzyl]-2,3-dihydro-1H-indole;
le 3,3-diméthyl-2-phényl-2-[4-(2-pipéridin-1-yléthoxy)-benzyl]-2,3-dihydro-1H-indol-5-ol;
le 1-[4-(2-azépan-1-yléthoxy)benzyl]-5-benzyloxy-3,3-diméthyl-2-phényl-2,3-dihydro-1H-indole;
le 1-[4-(2-azépan-1-yléthoxy)benzyl]-3,3-diméthyl-2-phényl-2,3-dihydro-1H-indol-5-ol,
le 5-benzyloxy-2-(4-benzyloxyphényl)-3,3-diméthyl-1-[4-(2-pipéridin-1-yléthoxy)benzyl]-2,3-dihydro-1H-indole;
le 2-(4-hydroxyphényl)-3,3-diméthyl-1-[4-(2-pipéridin-1-yléthoxy)benzyl]-2,3-dihydro-1H-indol-5-ol;
le 5-benzyloxy-2-(4-benzyloxyphényl)-3,3-diméthyl-1-[4-(2-azépan-1-yléthoxy)benzyl]-2,3-dihydro-1H-indole;

ou

le 1-[4-(2-azépan-1-yléthoxy)benzyl]-2-(4-hydroxy-phényl)-3,3-diméthyl-2-phényl-2,3-dihydro-1H-indol-5-ol,

ou un sel pharmaceutiquement acceptable de ceux-ci.

5. Composition pharmaceutique comprenant un composé suivant l'une quelconque des revendications 1 à 4, ou un sel pharmaceutiquement acceptable de celui-ci, et un vecteur ou excipient pharmaceutiquement acceptable.

6. Utilisation d'un composé suivant l'une quelconque des revendications 1 à 4 dans la préparation d'un médicament pour le traitement ou la prévention d'une perte osseuse chez un mammifère, le procédé comprenant une administration à un mammifère.

7. Utilisation d'un composé suivant l'une quelconque des revendications 1 à 4 dans la préparation d'un médicament pour le traitement ou la prévention d'états maladifs ou de syndromes qui sont provoqués par une déficience en oestrogènes ou associés à une telle déficience chez un mammifère.

8. Utilisation d'un composé suivant l'une quelconque des revendications 1 à 4 dans la préparation d'un médicament pour le traitement ou la prévention d'une maladie cardiovasculaire chez un mammifère, le procédé comprenant une administration à un mammifère.

9. Procédé de préparation d'un composé de formule I suivant la revendication 1 ou d'un sel pharmaceutiquement acceptable de celui-ci qui comprend l'un des suivants :

a) l'alkylation d'un composé de formule :

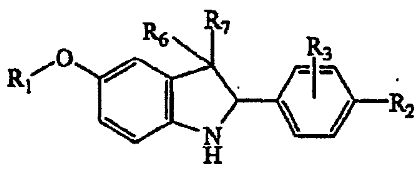

(II)

dans laquelle $R_1$, $R_2$, $R_3$, $R_6$ et $R_7$ sont tels que définis à la revendication 1, avec un composé de formule :

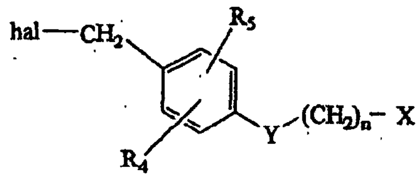

(III)

dans laquelle n, $R_4$, $R_5$, Y et X sont tels que définis à la revendication 1, pour donner un composé de formule I; ou

b) la réaction d'un composé de formule :

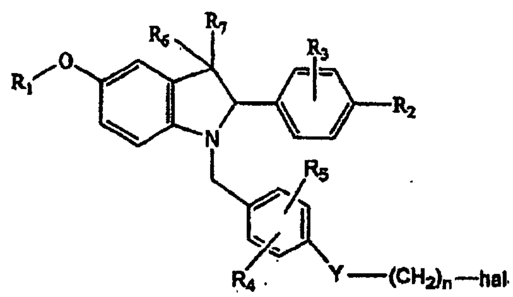

(IV)

dans laquelle Y, $R_1$, $R_2$, $R_3$, $R_4$, $R_5$, $R_6$ et $R_7$ sont tels que définis à la revendication 1 et hal représente un halogène, par exemple, un chlore ou un brome, avec une amine de formule :

H-X

(V)

dans laquelle X est tel que défini ci-dessus, pour donner un composé de formule I,

ou

c) l'élimination du benzyle dans un composé de formule I dans laquelle $R_1$ est facultativement substitué par un benzyle et/ou $R_2$ est facultativement substitué par un benzyloxy, pour donner un composé de formule I dans laquelle $R_1$ est un hydrogène et/ou $R_2$ est un hydroxy,

ou

e) l'estérification d'un composé de formule I dans laquelle au moins un parmi $R_3$, $R_4$ ou $R_5$ est un hydroxy en un dérivé ester de celui-ci.

**10.** Composé suivant l'une quelconque des revendications 1 à 4 pour une utilisation comme agent pharmaceutique.